(19) Europäisches Patentamt
European Patent Office
Office européen des brevets

(11) Veröffentlichungsnummer: **0 294 642 B1**

(12) **EUROPÄISCHE PATENTSCHRIFT**

(45) Veröffentlichungstag der Patentschrift: **03.03.93**

(51) Int. Cl.$^5$: **C08G 77/38**, A61K 7/06

(21) Anmeldenummer: **88108254.9**

(22) Anmeldetag: **24.05.88**

Die Akte enthält technische Angaben, die nach dem Eingang der Anmeldung eingereicht wurden und die nicht in dieser Patentschrift enthalten sind.

# (54) Diquaternäre Polysiloxane, deren Herstellung und Verwendung in kosmetischen Zubereitungen.

(30) Priorität: **06.06.87 DE 3719086**

(43) Veröffentlichungstag der Anmeldung:
**14.12.88 Patentblatt 88/50**

(45) Bekanntmachung des Hinweises auf die Patenterteilung:
**03.03.93 Patentblatt 93/09**

(84) Benannte Vertragsstaaten:
**BE DE ES FR GB IT NL**

(56) Entgegenhaltungen:
**EP-A- 17 121**
**EP-A- 0 017 122**
**BE-A- 666 745**
**FR-A- 2 514 014**
**US-A- 4 185 087**

(73) Patentinhaber: **Th. Goldschmidt AG**
**Goldschmidtstrasse 100 Postfach 101461**
**W-4300 Essen 1(DE)**

(72) Erfinder: **Schaefer, Dietmar, Dr.**
**Milchstrasse 40**
**W-4300 Essen 14(DE)**
Erfinder: **Krakenberg, Manfred**
**Meistersingerstrasse 39**
**W-4300 Essen 13(DE)**

**EP 0 294 642 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue diquaternäre Polysiloxane, wobie die quaternären Stickstoffgruppen am Polysiloxanmolekül endständig gebunden sind. Die Erfindung betrifft ferner die Herstellung dieser Verbindungen sowie deren Verwendung in kosmetischen Zubereitungen, insbesondere in Zubereitungen zur Pflege der Haare.

Es ist bekannt, Organopolysiloxane zur Herstellung von Haarpflegemitteln zu verwenden. In "Chemie und Technologie der Silicone" von Walter Noll, Verlag Chemie, 2. Auflage, 1968, Seite 536, heißt es allerdings, daß die Aufgabe, die Frisur unabhängig von Feuchtigkeitseinflüssen zu erhalten, mit normalen Polydimethylsiloxanolen nicht zu lösen sei. Das Silicon müsse vielmehr mit Hilfe von funktionellen Gruppierungen auf dem Haar fixiert werden.

Aus der DE-AS 14 93 384 sind Organosiloxanverbindungen oder -verbindungsgemische der Formel

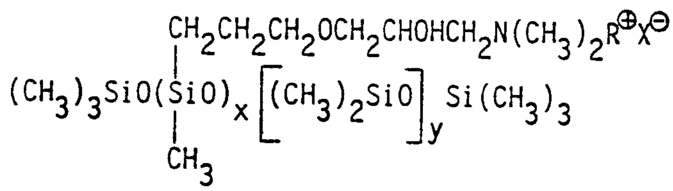

in der R für Wasserstoff oder $CH_3$ und X für Halogen steht und x = 1 bis 10 und y = 0 bis 8,5 sind, wobei y : x nicht größer als 8,5 : 1 ist, bekannt.

Diese Organosiloxane mit quaternären Ammoniumgruppen können dadurch hergestellt werden, daß man ein Epoxysiloxan der Formel

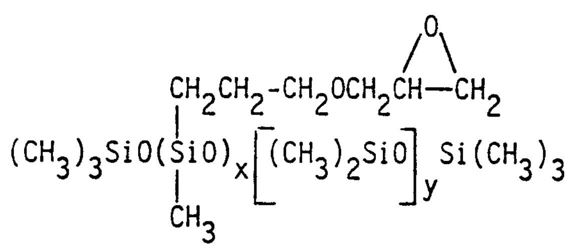

auf an sich bekannte Weise mit Dimethylamin umsetzt und das erhaltene Dimethylaminoorganosiloxan der Formel

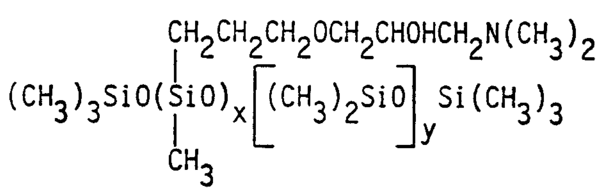

in an sich bekannter Weise mit einem Halogenwasserstoff oder mit einem Methylhalogenid in die quaternäre Ammoniumverbindung der vorgenannten Formel überführt.

Die vorgenannten Organopolysiloxane mit quaternären Ammoniumgruppen können entsprechend der US-PS 4 185 087 für Haarpflegemittel verwendet werden. Dort ist ausgeführt, daß zwar einfache wäßrige Haarwaschmittel das Haar von Schmutz befreien und einen Überschuß an Fett entfernen könnten. Bei den meisten Haarwaschmitteln würde jedoch die Entfettung des Haares so gründlich vorgenommen, daß eine Schädigung des Haares zu beobachten sei. Die Haare würden sich nach der Wäsche elektrostatisch aufladen und deshalb schlecht kämmbar sein.

Der Zusatz von Lanolinderivaten, Glykol, Fettsäureestern oder Proteinen verbessere zwar die Handhabbarkeit des Haares nach dem Waschen, beeinträchtige aber gleichzeitig die Schaumbildung. Die Haare würden etwa klebrig und fühlten sich unnatürlich an.

Die vorbeschriebenen Organopolysiloxane mit quaternären Ammoniumgruppen sollen nach den Angaben der US-PS 4 185 087 diese Nachteile beseitigen und die Kämmbarkeit der gewaschenen Haare, den Halt der Frisur, den Glanz der Haare verbessern.

Für die Herstellung der in der DE-AS 14 93 384 beschriebenen Verbindungen geht man von den entsprechenden Methylwasserstoffpolysiloxanen aus. Diese sind im allgemeinen äquilibrierte Gemische, d.h. Siloxangemische, bei denen die Anzahl der Methylwasserstoffsiloxy- und Dimethylsiloxyeinheiten einer statistischen Verteilung entsprechen. Bei Siloxanen mit niedrigem Wert von x sind deshalb stets nicht zu vernachlässigende Anteile an solchen Siloxanen vorhanden, bei denen x = 0 ist. Im Verfahrensendprodukt ist deshalb ein Anteil an unmodifizierten Siliconölen nicht zu vermeiden. Dieser Anteil trägt jedoch nicht zur Verbesserung der Kämmbarkeit des Haares, der Frisur und des Glanzes des Haares bei.

Ein weiterer Nachteil der in der DE-AS-14 93 384 beschriebenen Verbindungen besteht ferner darin, daß die Dimethylsiloxyketten stets durch Methylsiloxygruppen unterbrochen werden, welche seitenständig quaternäre Stickstoffgruppen aufweisen. Der typische Siloxancharakter, der zur Verbesserung der Eigenschaften des Haares erwünscht ist, beruht aber gerade auf der Anwesenheit von Dimethylsiloxyketten. Die Kämmbarkeit und der Glanz des Haares sind deshalb nicht optimal gewährleistet.

Eine ähnliche Lehre ergibt sich aus der EU-PS 0 017 121 (entsprechend DE-OS 29 12 485). Auch hier sind Organopolysiloxane mit quaternären Ammoniumgruppen in Haarwasch- und Haarbehandlungsmitteln zur Verbesserung der frisiertechnischen Eigenschaften der Haare beschrieben. Die Verbindungen entsprechen dabei der allgemeinen Formel

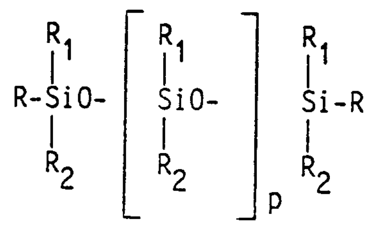

in der $R_1$ und $R_2$ einen Alkylrest mit 1 bis 4 Kohlenstoffatomen oder einen Arylrest, p die Zahlen 0 bis 50 und R die Reste

a) 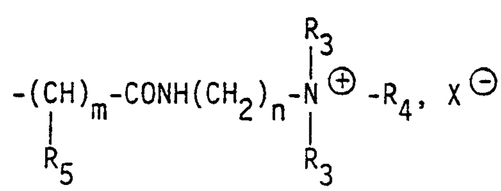

oder

b) 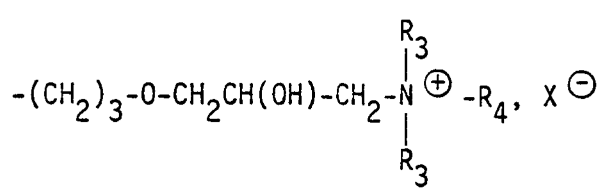

darstellen, wobei $R_3$ einen Alkyl- oder Hydroxy-alkylrest mit 1 bis 3 Kohlenstoffatomen, $R_4$ einen Rest gleich $R_3$ oder Aryl-$CH_2$- oder den Allylrest, $R_5$ Wasserstoff oder den Methylrest, $X^{\ominus}$ die Anionen $Cl^{\ominus}$, $Br^{\ominus}$, $J^{\ominus}$, $CH_3SO_4{}^{\ominus}$ oder $C_2H_5SO_4{}^{\ominus}$ und m die Zahlen 2 bis 10, n die Zahlen 2 bis 4 bedeuten.

Auch die Verbindungen, welche in der EU-PS 0 017 121 beschrieben sind, können bei ihrer Anwendung in Zubereitungen zur Pflege der Haare noch nicht völlig befriedigen. Beispielsweise ist die Kämmbarkeit von Haaren, welche mit den Verbindungen der EU-PS 0 017 121 behandelt worden sind, noch nicht ausreichend. Dies ist insbesondere dann zu beobachten, wenn das Molekulargewicht dieser Verbindungen bei kleinen Werten von p niedrig ist. Die Verbindungen können dann auch leicht ausgewaschen werden.

Hinzu kommt, daß die einem Abbau dieser Verbindungen, z.B. thermischer Zersetzung, Amine mit niedrigen Alkylresten frei werden, welche einen unangenehmen Geruch haben.

Der vorliegenden Erfindung liegt die Aufgabe zugrunde, Verbindungen aufzufinden, welche verbesserte Gebrauchseigenschaften aufweisen und insbesondere die Pflege der Haare verbessern.

Überraschenderweise wurde nun gefunden, daß dieses gewünschte Eigenschaftsprofil bei neuen Verbindungen der allgemeinen Formel

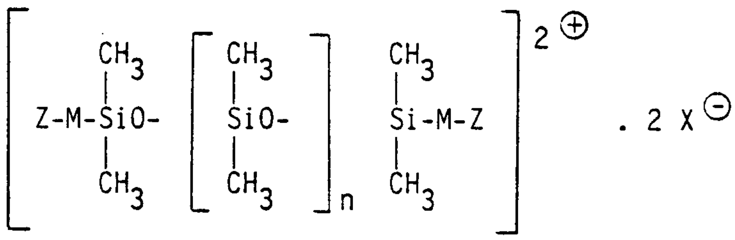

wobei
Z der Rest

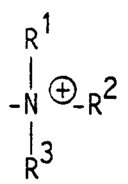 oder 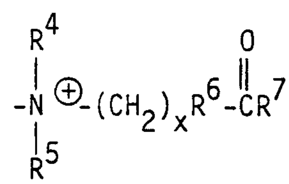 ist,

| | | |
|---|---|---|
| $R^1$,$R^2$, $R^3$ | = | Alkylreste mit 1 bis 2 Kohlenstoffatomen oder Alkenylreste mit 2 bis 22 Kohlenstoffatomen, wobei mindestens einer der Reste $R^1$, $R^2$, $R^3$ mindestens 10 Kohlenstoffatome aufweist, |
| $R^4$, $R^5$, $R^7$ | = | Alkylreste mit 1 bis 22 Kohlenstoffatomen oder Alkenylreste mit 2 bis 22 Kohlenstoffatomen, |
| $R^6$ | = | -O- oder -$NR^8$-Rest, $R^8$ = Alkyl- oder Hydroxyalkylrest mit 1 bis 4 Kohlenstoffatomen oder Wasserstoffrest, |
| x | = | 2 bis 4, |
| M | = | ein zweiwertiger Kohlenwasserstoffrest mit mindestens 4 Kohlenstoffatomen, der eine Hydroxylgruppe aufweist und der durch ein Sauerstoffatom unterbrochen sein kann, |
| n | = | eine Zahl 0 bis 200, |
| $X^{\ominus}$ | = | anorganisches oder organisches Anion. |

Die Reste $R^1$, $R^2$, $R^3$ sowie die Reste $R^4$, $R^5$ und $R^7$ können im Molekül gleich oder verschieden sein. Bevorzugt sind solche Verbindungen, bei denen zwei der Reste $R^1$, $R^2$ und $R^3$ niedere Alkylreste mit 1 bis 4 Kohlenstoffatomen sind und der verbleibende dritte Rest ein langkettiger Kohlenwasserstoffrest mit mindestens 10 Kohlenstoffatomen ist. An langkettigen Kohlenwasserstoffresten sind insbesondere die gesätligten und ungesättigten Kohlenwasserstoffreste bevorzugt, welche sich von den natürlich vorkommenden Fettsäuren herleiten.

Liegen die Reste $R^4$, $R^5$ und $R^7$ nebeneinander vor, sind diejenigen Verbindungen bevorzugt, bei denen $R^4$ und $R^5$ einen niederen Kohlenwasserstoffrest mit 1 bis 4 Kohlenstoffatomen bedeuten und der Rest $R^7$ von einer Fettsäure $R^7COOH$ abgeleitet ist, die mindestens 8 Kohlenstoffatome aufweist.

Innerhalb der erfindungsgemäßen Verbindungen können die beiden Reste Z gleiche oder unterschiedliche Bedeutung haben.

M ist ein zweiwertiger Kohlenwasserstoffrest mit mindestens 4 Kohlenstoffatomen, der eine Hydroxylgruppe aufweist und der durch ein Sauerstoffatom unterbrochen sein kann. Besonders bevorzugt sind die folgenden Reste

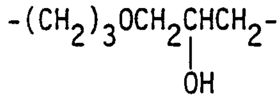 , 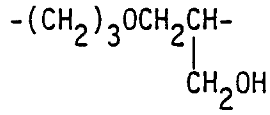 ,

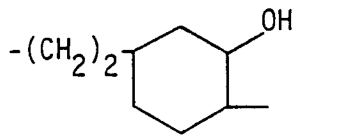 , 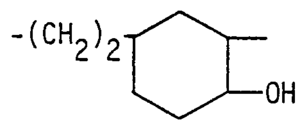 ,

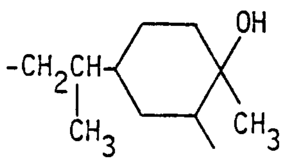 , 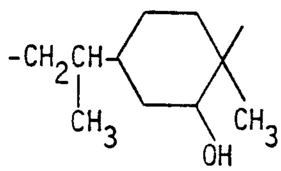

Beispiele weiterer Reste M sind

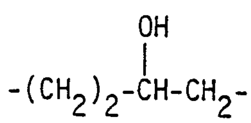 , 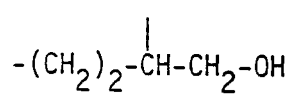 ,

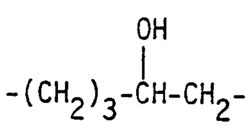 , 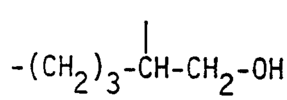 ,

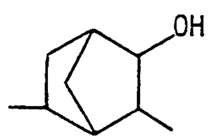 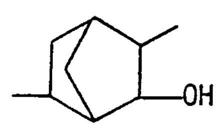

Der Index n ist eine Zahl von 0 bis 200. Hat der Index n den Wert von 0, liegt ein Disiloxan mit zwei quaternären Endgruppe vor. Bei höheren Werten von n ist es herstellungsbedingt, daß die Verbindungen in Form von Gemischen vorliegen, bei denen n dann als Mittelwert zu verstehen ist.

$X^{\ominus}$ ist ein anorganisches oder organisches Anion. Bei Verwendung der erfindungsgemäßen neuen Polymeren als Bestandteil von Haarpflegemitteln ist darauf zu achten, daß das Anion von einer physiologisch verträglichen Säure herrührt. Beispiele geeigneter Anionen sind Acetationen, Chloridionen, Bromidionen, Hydrogensulfationen, Sulfationen.

Bezeichnet man die quaternären Stickstoffgruppen mit A und die Polysiloxanblöcke mit B handelt es sich bei den erfindungsgemäßen Verbindungen um polymere Verbindungen der Struktur ABA.

Beispiele erfindungsgemäßer diquaternärer Siloxane sind

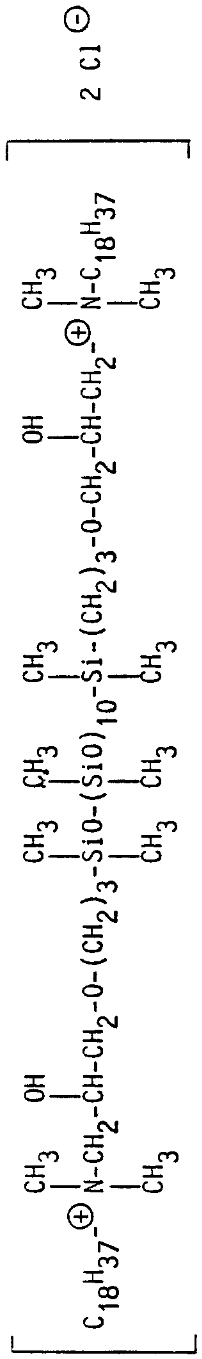

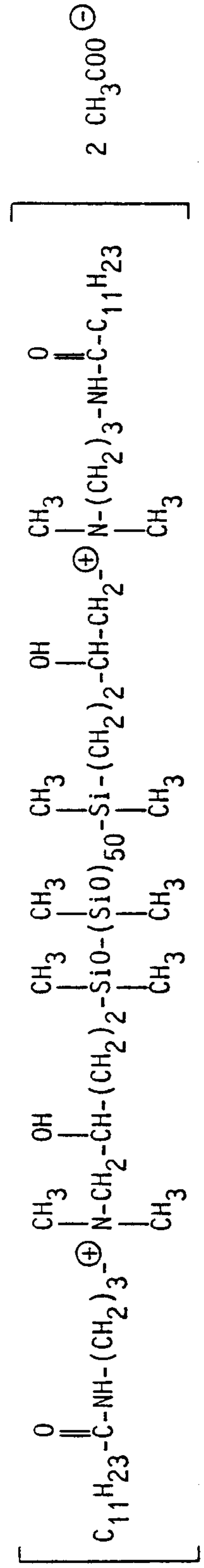

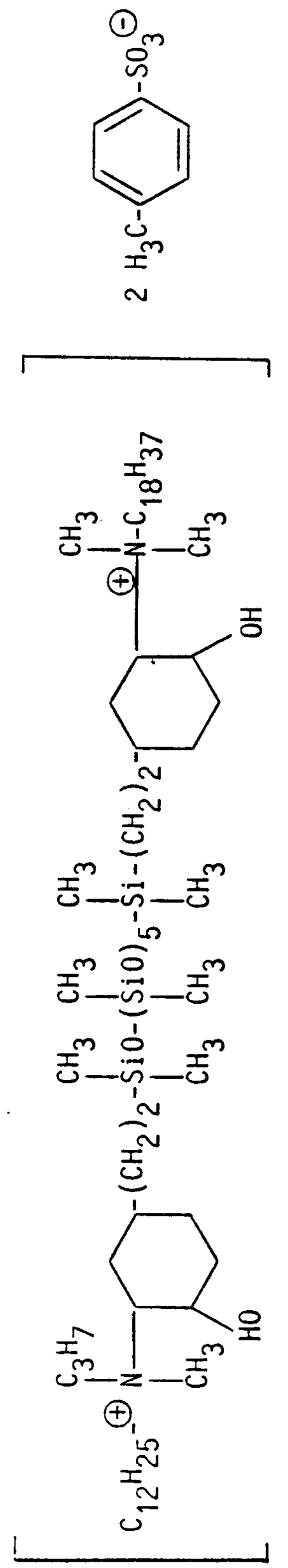

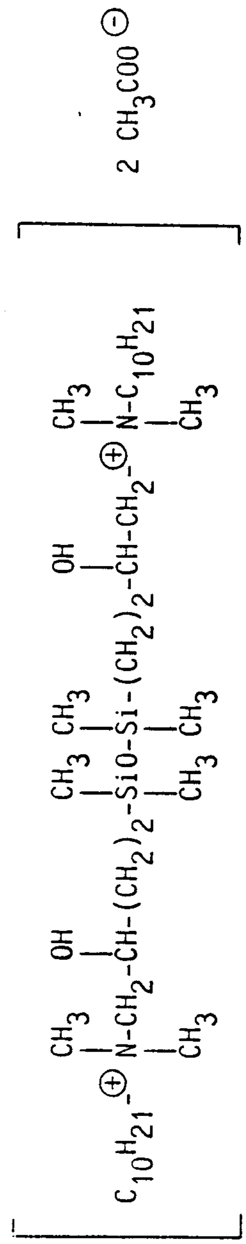

Die erfindungsgemäßen Produkte sind viskose bis hochviskose, ölige bis pastenartige, farblose bis schwach gelb oder rötlich gefärbte Produkte. Die Löslichkeit der erfindungsgemäßen Polymeren wird durch das Verhältnis der Dimethylsiloxyeinheiten und der Zahl der quaternären Ammoniumgruppen und durch das Molekulargewicht der Verbindungen bestimmt.

Für die Anwendung in der Kosmetik, speziell in Haarpflegemittel, sind im allgemeinen Produkte bevorzugte, die in Wasser oder in wassermischbaren Hilfslösungsmitteln, wie ein- oder mehrwertigen

Alkoholen, löslich oder dispergierbar sind.

Ein weiterer Gegenstand der Erfindung besteht in der Herstellung der neuen Verbindungen, die in analoger Weise entsprechend dem Stand der Technik erfolgt. Das Verfahren ist dadurch gekennzeichnet, daß man Verbindungen der allgemeinen Formel

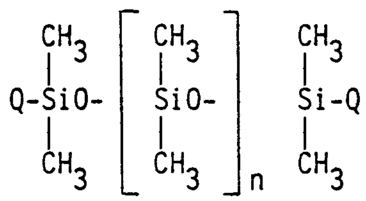

II

wobei

Q ein Kohlenwasserstoffrest mit mindestens 4 Kohlenstoffatomen, der eine Epoxidgruppe aufweist und durch ein Sauerstoffatom unterbrochen sein kann,

n wie oben definiert ist,

mit tertiären Aminen der allgemeinen Formel

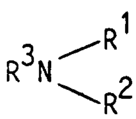 oder 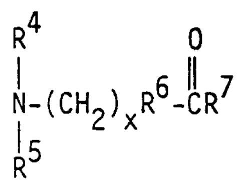 ,

wobei $R^1$, $R^2$, $R^3$, $R^4$, $R^5$, $R^6$, $R^7$ und x oben definert sind, in an sich bekannter Weise in solchen Mengenverhältnissen umsetzt, daß jeder Epoxidgruppe mindestens eine tertiäre Aminogruppe entspricht, und man die Umsetzung in Gegenwart eines Säureäquivalentes HX, bezogen auf zu quaternierendes Stickstoffatom, und bei Temperaturen von 40 bis 120 °C durchführt.

Q ist ein Kohlenwasserstoffrest mit mindestens 4 Kohlenstoffatomen, der eine Epoxidgruppe aufweist und durch ein Sauerstoffatom unterbrochen sein kann. Bevorzugte Beispiele solcher Gruppen Q sind die Gruppen

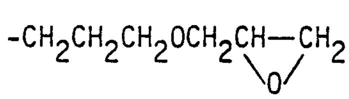 , 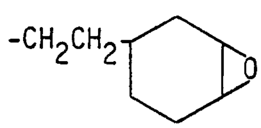 , 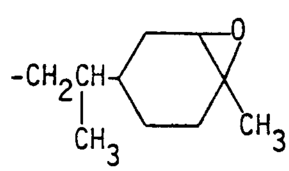

Der Index n hat die bereits angegebene Bedeutung.

Die Polysiloxane mit endständigen Epoxidgruppen der Formel II werden mit den vorgenannten tertiären Aminen in an sich bekannter Weise umgesetzt. Die Reaktion wird in Gegenwart eines Säureäquivalentes HX, bezogen auf zu quaternierendes Stickstoffatom, durchgeführt. Dabei werden solche Mengenverhältnisse eingehalten, daß jeder Gruppe Q der Verbindung II mindestens eine tertiäre Aminogruppe entspricht. Die Umsetzung erfolgt vorzugsweise in wäßriger oder wäßrig/alkoholischer Lösung. Zur Beschleunigung der Umsetzung arbeitet man zweckmäßig bei erhöhten Temperaturen, wobei die maximale Umsetzungstemperatur im allgemeinen durch die Rückflußtemperatur des Lösungsmittels gegeben ist. Bevorzugt ist deshalb eine Umsetzungstemperatur von 40 bis 120 °C.

Ein weiterer Gegenstand der Erfindung liegt in der Verwendung der erfindungsgemäßen Verbindungen in der Kosmetik, insbesondere, wie eingangs ausgeführt, in der Verwendung der Verbindungen in Zubereitungen zur Pflege der Haare. Die erfindungsgemäßen Verbindungen weisen die geforderte Kombination der Eigenschaften, die eingangs aufgezählt worden sind, auf.

Verfahren zur Herstellung der erfindungsgemäßen Verbindungen und die Eigenschaften dieser Verbindungen sind in den folgenden Beispielen näher beschrieben.

Beispiel 1

Herstellung eines erfindungsgemäßen diquaternären Polysiloxans der allgemeinen Formel

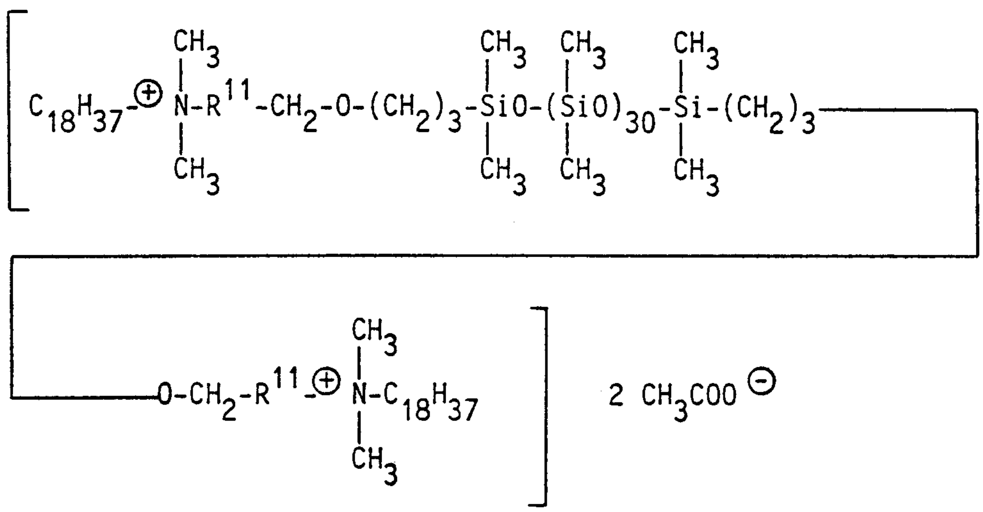

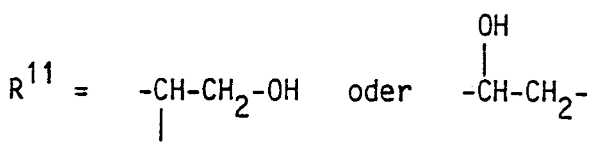

In einem 1-l-Vierhalskolben, versehen mit Rührer, Tropftrichter, Thermometer und Rückflußkühler, werden 59,4 g (0,2 Mol) Stearyldimethylamin mit 80 g $H_2O$ vorgelegt und bei 20 °C mit 12 g (0,2 Mol) Essigsäure versetzt. Nach 30 Minuten wird auf 50 °C erwärmt, anschließend werden 258,2 g (0,1 Mol) eines Epoxysiloxans der allgemeinen Formel

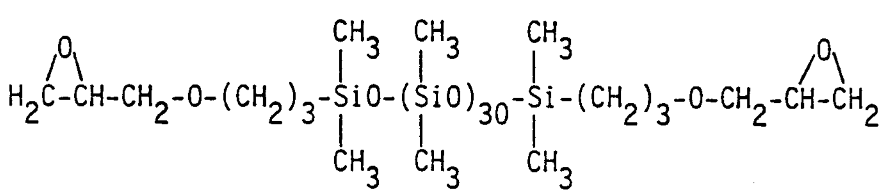

zugetropft. Nach Zugabe von 200 ml Isopropanol wird auf Rückflußtemperatur erwärmt und 6 Stunden gerührt. Das Wasser/Isopropanol-Gemisch wird bei 100 °C und 0,2 bar abdestilliert.

Erhalten: 325 g diquaternäres Polysiloxan; gelbes bis rötlich gefärbtes Produkt, bei Raumtemperatur hochviskos.

Quaternärer Stickstoff gef.: 0,8% theor.: 0,8 %.

Beispiel 2

Herstellung eines erfindungsgemäßen diquaternären Polysiloxans der allgemeinen Formel

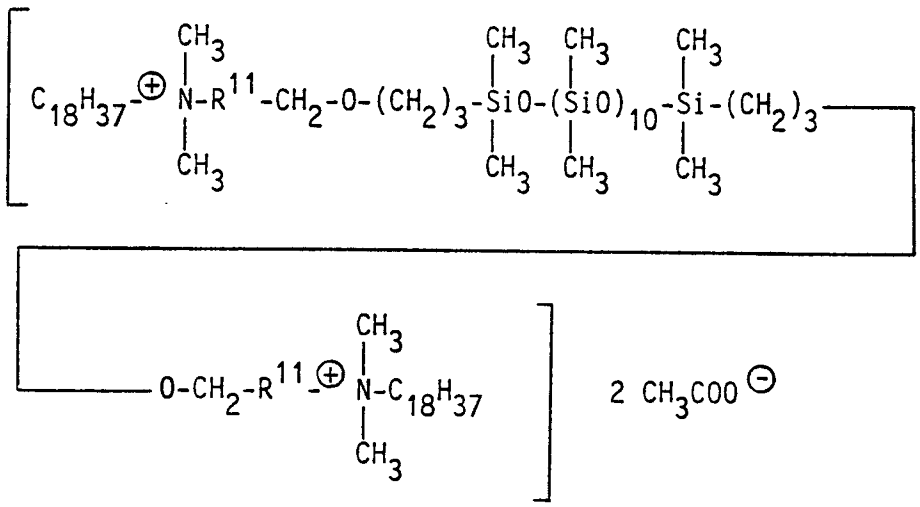

Durchführung wie in Beispiel 1 beschrieben mit einem Epoxysiloxan, dessen Wert von n = 10 ist.

| Ansatz: | |
|---|---|
| Stearyldimethylamin: | 59,4 g (0,2 Mol) |
| Essigsäure: | 12,0 g (0,2 Mol) |
| Epoxysiloxan *): | 110,2 g (0,1 Mol) |
| Wasser: | 80 g |
| Isopropanol: | 200 g |

*) Epoxysiloxan der allgemeinen Formel:

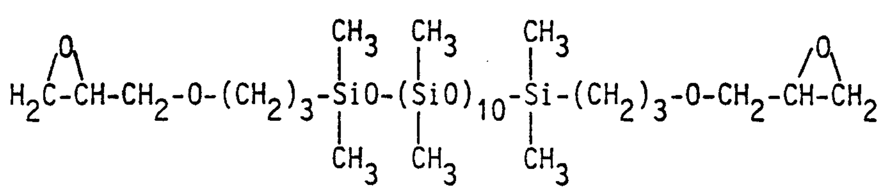

Erhalten: 176 g diquaternäres Polysiloxan; gelbes bis rötlich gefärbtes Produkt, bei Raumtemperatur hochviskos.

Quaternärer Stickstoff gef.: 1,4 % theor.: 1,5 %.

Beispiel 3

Herstellung eines erfindungsgemäßen diquaternären Polysiloxans der allgemeinen Formel

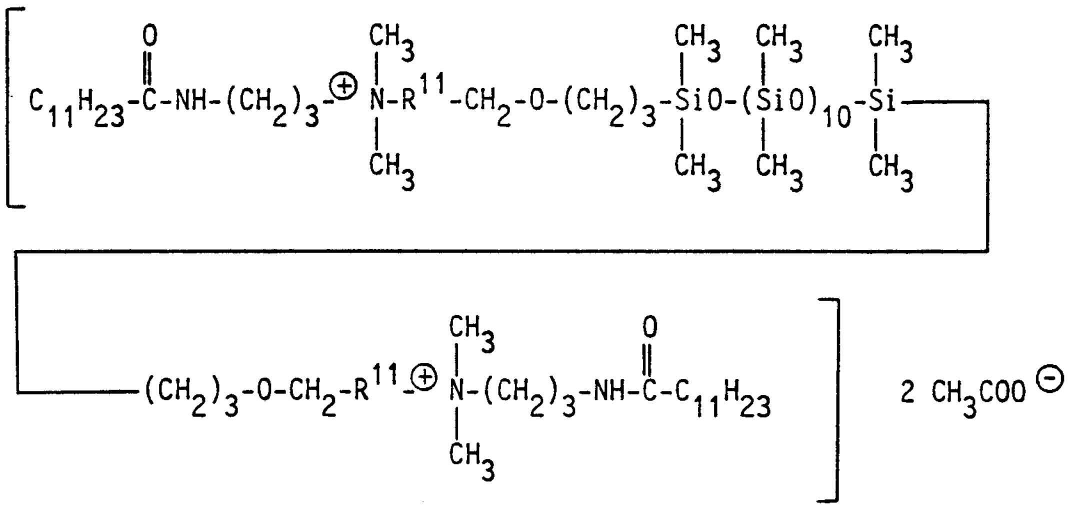

Durchführung wie in Beispiel 1 beschrieben, jedoch mit Lauryl(dimethylaminopropyl)amid anstelle von Stearyldimethylamin und dem Epoxysiloxan mit n = 10 aus Beispiel 2.

| Ansatz: | |
|---|---|
| Lauryl(dimethylaminopropyl)amid: | 56,8 g (0,2 Mol) |
| Essigsäure: | 12,0 g (0,2 Mol) |
| Epoxysiloxan aus Beispiel 2: | 110,2 g (0,1 Mol) |
| Wasser: | 80 g |
| Isopropanol: | 200 g |

Erhalten: 174 g diquaternäres Polysiloxan; gelbes bis rötlich gefärbtes Produkt, bei Raumtemperatur hochviskos.

Quaternärer Stickstoff gef.: 1,5 % theor.: 1,6 %.

Beispiel 4

Herstellung und Überprüfung von Haarbehandlungsmitteln unter Verwendung der in den Beispielen 1 bis 3 hergestellten diquaternären Polysiloxane (%-Angaben = Gew.-%)

Konditioniershampoo

| | | |
|---|---|---|
| A | Tego®-Betain L 7 1): | 2 % |
| | Antil®141 Liquid 2): | 3 % |
| B | Si-Quat aus Beispiel 1: | 2 % |
| | 1,2-Propylenglykol: | 4 % |
| C | Natriumlaurylethersulfat: | 10 % |
| D | Wasser: | 79 % |

Zur Herstellung werden die Bestandteile in der aufgeführten Reihenfolge (A→E) zusammengegeben. Jede Mischung muß vor Zugabe weiterer Komponenten klar gelöst sein.

| | Cremespülung I | | Cremespülung II | |
|---|---|---|---|---|
| A | Teginacid®X 3): | 6 % | Teginacid®X 3): | 6 % |
| | Cetylalkohol: | 0,5 % | Cetylalkohol: | 0,5 % |
| B | Si-Quat aus Beispiel 2: | 2 % | Si-Quat aus Beispiel 3: | 2 % |
| | Wasser: | 91,5 % | Wasser: | 91,5 % |

Zur Herstellung werden A und B zusammengegeben, homogenisiert und unter Rühren abgekühlt.

1) Tego®-Betain L 7 = Cocamidopropyl-Betain (1-Alkoylamino-3-dimethylammonium-propan-3-carboxymethyl-betain)

2) Antil®141 Liquid ist ein flüssiges Verdickungsmittel auf der Basis eines nichtionogenen Fettsäurepolyalkylenglykolesters

3) Teginacid®X ist ein O/W-Emulgator auf der Basis einer Mischung von Glycerinmono/distearaten mit Polyglykolfettalkoholethern

In der praktischen Anwendung beim halbseitigen Vergleichstest auf menschlichem Haar ergibt sich gegenüber Shampooformulierungen bzw. Cremespülungen mit quaternären Siloxanen aus dem Stand der Technik sowie gegenüber Konditioniershampoos und Cremespülungen des Marktes auf Basis rein organischer Quats eine verbessert Naß- und Trockenkämmbarkeit sowie verbesserter Glanz der behandelten Haare.

## Patentansprüche

**Patentansprüche für folgende Vertragsstaaten : BE, DE, FR, GB, IT, NL**

**1.** Verbindungen der allgemeinen Formel

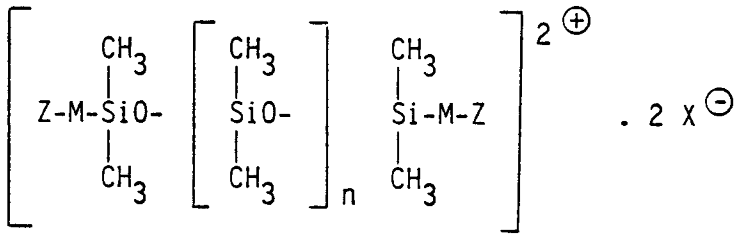

wobei
Z der Rest

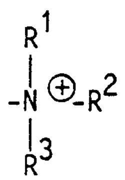

oder

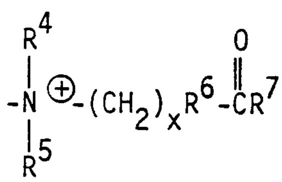

ist,

| | | |
|---|---|---|
| $R^1$,$R^2$, $R^3$ | = Alkylreste mit 1 bis 22 Kohlenstoffatomen oder Alkenylreste mit 2 bis 22 Kohlenstoffatomen, wobei mindestens einer der Reste $R^1$, $R^2$, $R^3$ mindestens 10 Kohlenstoffatome aufweist, |
| $R^4$, $R^5$, $R^7$ | = Alkylreste mit 1 bis 22 Kohlenstoffatomen oder Alkenylreste mit 2 bis 22 Kohlenstoffatomen, |
| $R^6$ | = -O- oder -$NR^8$-Rest, $R^8$ = Alkyl- oder Hydroxyalkylrest mit 1 bis 4 Kohlenstoffatomen oder Wasserstoffrest, |
| x | = 2 bis 4, |
| M | = ein zweiwertiger Kohlenwasserstoffrest mit mindestens 4 Kohlenstoffatomen, der eine Hydroxylgruppe aufweist und der durch ein Sauerstoffatom unterbrochen sein kann, |
| n | = eine Zahl von 0 bis 200, |
| $X^{\ominus}$ | = anorganisches oder organisches Anion. |

**2.** Verfahren zur Herstellung von Verbindungen des Anspruchs 1, dadurch gekennzeichnet, daß man Verbindungen der allgemeinen Formel

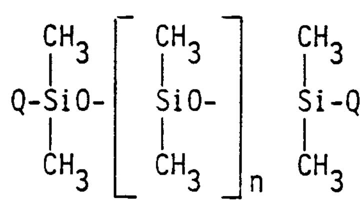

wobei

Q ein Kohlenwasserstoffrest mit mindestens 4 Kohlenstoffatomen, der eine Epoxidgruppe aufweist und durch ein Sauerstoffatom unterbrochen sein kann,

n wie oben definiert ist,

mit tertiären Aminen der allgemeinen Formel

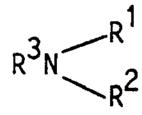 oder 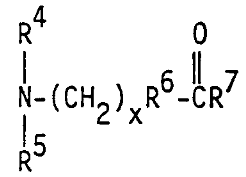 ,

wobei $R^1$, $R^2$, $R^3$, $R^4$, $R^5$, $R^6$, $R^7$ und x wie oben definiert sind, in an sich bekannter Weise in solchen Mengenverhältnissen umsetzt, daß jeder Epoxidgruppe mindestens eine tertiäre Aminogruppe entspricht, und man die Umsetzung in Gegenwart eines Säureäquivalentes HX, bezogen auf zu quaternierendes Stickstoffatom, und bei Temperaturen von 40 bis 120 ° C durchführt.

**3.** Verwendung der Verbindungen des Anspruchs 1 in kosmetischen Zubereitungen, insbesondere in Zubereitungen zur Pflege der Haare.

**Patentansprüche für folgenden Vertragsstaat : ES**

**1.** Verfahren zur Herstellung von Verbindungen der allgemeinen Formel

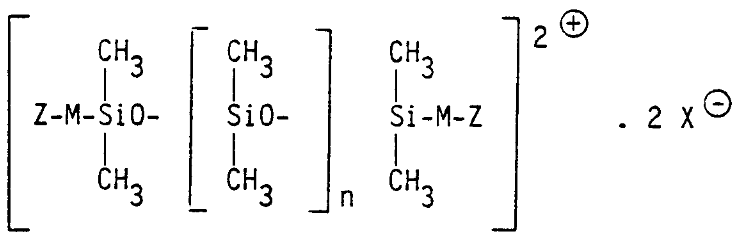

wobei
Z der Rest

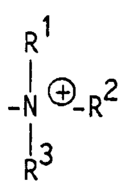 oder 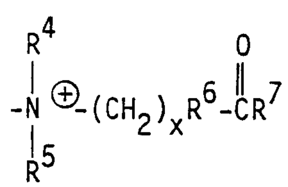 ist,

| | | |
|---|---|---|
| $R^1$,$R^2$, $R^3$ | = | Alkylreste mit 1 bis 22 Kohlenstoffatomen oder Alkenylreste mit 2 bis 22 Kohlenstoffatomen, wobei mindestens einer der Reste $R^1$, $R^2$, $R^3$ mindestens 10 Kohlenstoffatome aufweist, |
| $R^4$, $R^5$, $R^7$ | = | Alkylreste mit 1 bis 22 Kohlenstoffatomen oder Alkenylreste mit 2 bis 22 Kohlenstoffatomen, |
| $R^6$ | = | -O- oder -$NR^8$-Rest, $R^8$ = Alkyl- oder Hydroxyalkylrest mit 1 bis 4 Kohlenstoffatomen oder Wasserstoffrest, |
| x | = | 2 bis 4, |
| M | = | ein zweiwertiger Kohlenwasserstoffrest mit mindestens 4 Kohlenstoffatomen, der eine Hydroxylgruppe aufweist und der durch ein Sauerstoffatom unterbrochen sein kann, |
| n | = | eine Zahl 0 bis 200, |
| $X^{\ominus}$ | = | anorganisches oder organisches Anion, |

dadurch gekennzeichnet, daß man Verbindungen der allgemeinen Formel

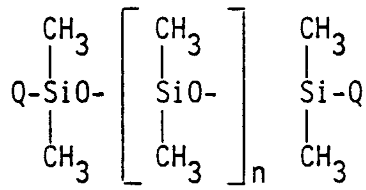

wobei

Q ein Kohlenwasserstoffrest mit mindestens 4 Kohlenstoffatomen, der eine Epoxidgruppe aufweist und durch ein Sauerstoffatom unterbrochen sein kann,

n wie oben definiert ist,

mit tertiären Aminen der allgemeinen Formel

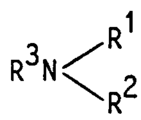

oder

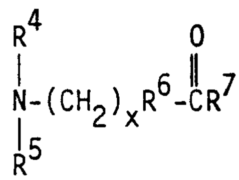

,

wobei $R^1$, $R^2$, $R^3$, $R^4$, $R^5$, $R^6$, $R^7$ und x oben definiert sind, in an sich bekannter Weise in solchen Mengenverhältnissen umsetzt, daß jeder Epoxidgruppe mindestens eine tertiäre Aminogruppe entspricht, und man die Umsetzung in Gegenwart eines Säureäquivalentes HX, bezogen auf zu quaternierendes Stickstoffatom, und bei Temperaturen von 40 bis 120 ° C durchführt.

**2.** Verwendung der Verbindungen des Anspruchs 1 in kosmetischen Zubereitungen, insbesondere in Zubereitungen zur Pflege der Haare.

**Claims**

**Claims for the following Contracting States : BE, DE, FR, GB, IT, NL**

**1.** Compounds of the general formula

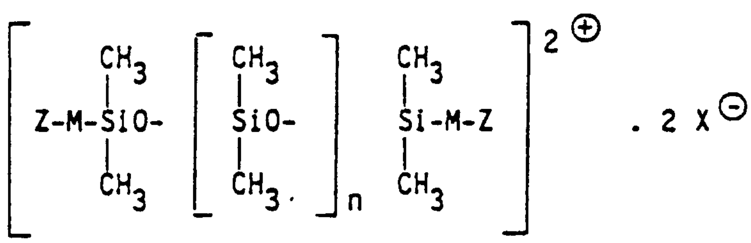

in which

Z is the radical

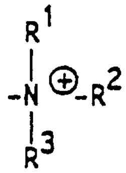

or

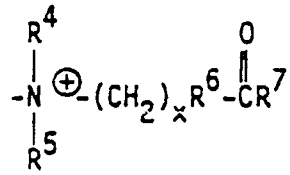

$R^1$, $R^2$, $R^3$ = alkyl radicals having 1 to 22 carbon atoms or alkenyl radicals having 2 to 22 carbon atoms, at least one of the radicals $R^1$, $R^2$, and $R^3$ having at least 10 carbon

atoms,

| | |
|---|---|
| $R^4$, $R^5$, $R^7$ | = alkyl radicals having 1 to 22 carbon atoms or alkenyl radicals having 2 to 22 carbon atoms, |
| $R^6$ | = -O- or $-NR^8-$ radical, $R^8$ = alkyl or hydroxyalkyl radical having 1 to 4 carbon atoms or hydrogen radical, |
| x | = 2 to 4, |
| M | = a divalent hydrocarbon radical having at least 4 carbon atoms, which has a hydroxyl group and which can be interrupted by an oxygen atom, |
| n | = a number from 0 to 200, and |
| $X^{\ominus}$ | = inorganic or organic anion. |

**2.** Process for the preparation of compounds of Claim 1, characterised in that compounds of the general formula

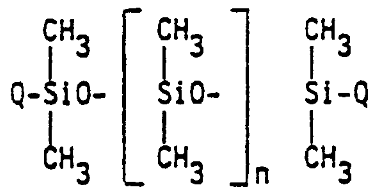

in which

Q a hydrocarbon radical having at least 4 carbon atoms, which has an epoxide group and can be interrupted by an oxygen atom, and

n is as defined above,

are reacted with tertiary amines of the general formula

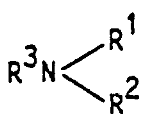

or

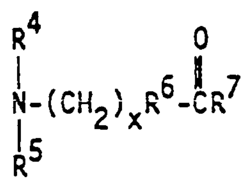

,

in which $R^1$, $R^2$, $R^3$, $R^4$, $R^5$, $R^6$, $R^7$ and x are as defined above, in a manner known per se in proportion such that each epoxide group corresponds to at least one tertiary amino group and the reaction is carried out in the presence of an acid equivalent HX, based on the nitrogen atom to be quaternised, and at temperatures of 40 to 120 °C.

**3.** Use of the compounds of Claim 1 in cosmetic formulations, in particular in formulations for hair care.

**Claims for the following Contracting State : ES**

**1.** Process for the preparation of compounds of the general formula

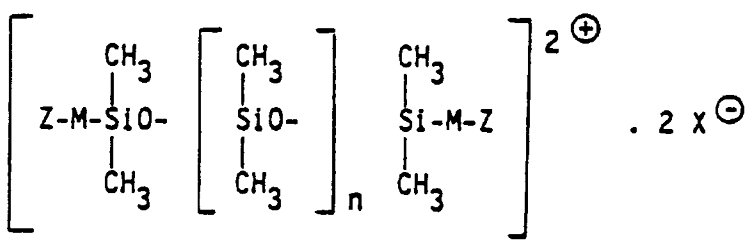

in which
Z is the radical

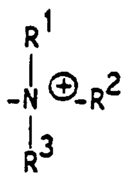

or

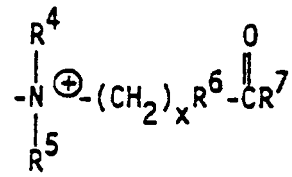

$R^1$, $R^2$, $R^3$ = alkyl radicals having 1 to 22 carbon atoms or alkenyl radicals having 2 to 22 carbon atoms, at least one of the radicals $R^1$, $R^2$, and $R^3$ having at least 10 carbon atoms,
$R^4$, $R^5$, $R^7$ = alkyl radicals having 1 to 22 carbon atoms or alkenyl radicals having 2 to 22 carbon atoms,
$R^6$ = -O- or -$NR^8$- radical, $R^8$ = alkyl or hydroxyalkyl radical having 1 to 4 carbon atoms or hydrogen radical,
x = 2 to 4,
M = a divalent hydrocarbon radical having at least 4 carbon atoms, which has a hydroxyl group and which can be interrupted by an oxygen atom,
n = a number from 0 to 200, and
$X^{\ominus}$ = inorganic or organic anion,

characterised in that compounds of the general formula

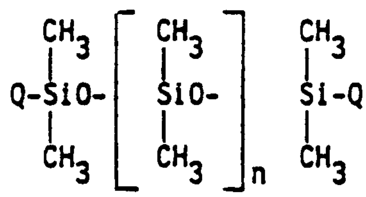

in which
Q a hydrocarbon radical having at least 4 carbon atoms, which has an epoxide group and can be interrupted by an oxygen atom, and
n is as defined above,

are reacted with tertiary amines of the general formula

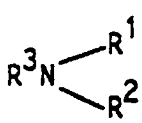

or

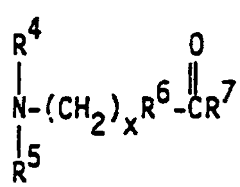

,

in which $R^1$, $R^2$, $R^3$, $R^4$, $R^5$, $R^6$, $R^7$ and x are as defined above, in a manner known per se in proportion such that each epoxide group corresponds to at least one tertiary amino group and the reaction is carried out in the presence of an acid equivalent HX, based on the nitrogen atom to be quaternised, and at temperatures of 40 to 120 ° C.

**2.** Use of the compounds of Claim 1 in cosmetic formulations, in particular in formulations for hair care.

**Revendications**

**Revendications pour les Etats contractants suivants : BE, DE, FR, GB, IT, NL**

**1.** Composés répondant à la formule générale :

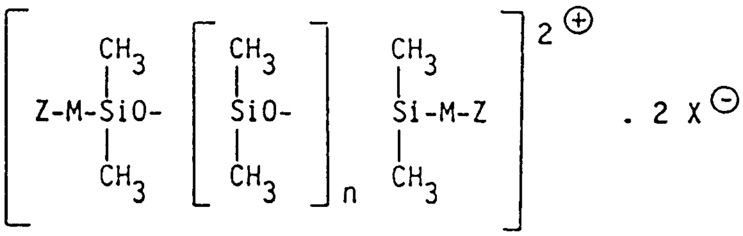

où

Z est le reste

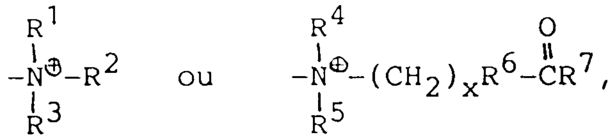

| | |
|---|---|
| $R^1$, $R^2$, $R^3$ | = des restes alcoyles comportant 1 à 22 atomes de carbone ou des restes alcényles comportant 2 à 22 atomes de carbone, au moins un des restes $R^1$, $R^2$, $R^3$ comportant au moins 10 atomes de carbone, |
| $R^4$, $R^5$, $R^7$ | = des restes alcoyles comportant 1 à 22 atomes de carbone ou des restes alcényles comportant 2 à 22 atomes de carbone, |
| $R^6$ | = -O- ou le reste $-NR^8$, $R^8$ = un reste alcoyle ou hydroxyalcoyle comportant 1 à 4 atomes de carbone, ou un reste d'hydrogène, |
| x | = 2 à 4, |
| M | = un reste hydrocarbure bivalent comportant au moins 4 atomes de carbone, qui possède un groupe hydroxyle et qui peut être interrompu par un atome d'oxygène, |
| n | = un nombre valant de 0 à 200, |
| $X^{\ominus}$ | = un anion inorganique ou organique. |

**2.** Procédé de préparation des composés selon la revendication 1, caractérisé en ce qu'on fait réagir des composés répondant à la formule générale :

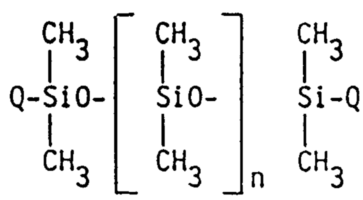

où

| | |
|---|---|
| Q | est un reste hydrocarbure comportant au moins 4 atomes de carbone, qui peut comporter un groupe époxyde et être interrompu par un atome d'oxygène, |
| n | est défini comme ci-dessus, |

avec des amines tertiaires répondant à la formule générale:

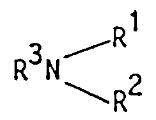

ou

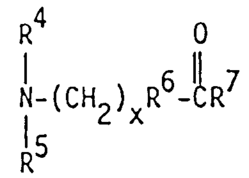

,

où $R^1$, $R^2$, $R^3$, $R^4$, $R^5$, $R^6$, $R^7$ et x sont définis comme ci-dessus, d'une manière connue en soi, en utilisant des rapports quantitatifs tels qu'à chaque groupe époxyde correspond au moins un groupe amino tertiaire, et on effectue la réaction en présence d'un équivalent d'acide HX, par rapport à l'atome d'azote à quaterniser, et à des températures comprises entre 40 et 120 ° C.

**3.** Utilisation des composes selon la revendication 1 dans des préparations cosmétiques, notamment dans des préparations pour le soin des cheveux.

**Revendications pour l'Etat contractant suivant : ES**

**1.** Procédé de préparation de composés répondant à la formule générale :

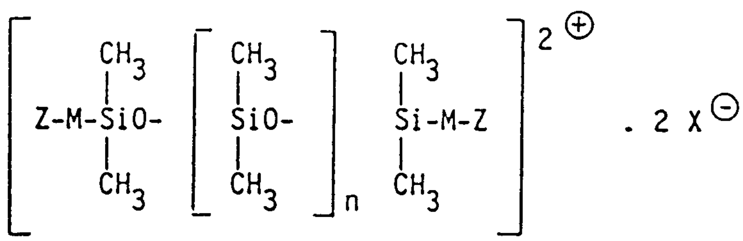

où
Z est le reste

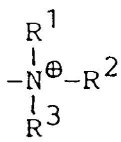

ou

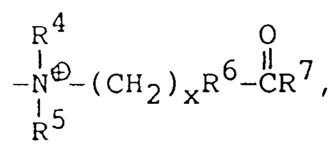

| | |
|---|---|
| $R^1$, $R^2$, $R^3$ | = des restes alcoyles comportant 1 à 22 atomes de carbone ou des restes alcényles comportant 2 à 22 atomes de carbone, au moins un des restes $R^1$, $R^2$, $R^3$ comportant au moins 10 atomes de carbone, |
| $R^4$, $R^5$, $R^7$ | = des restes alcoyles comportant 1 à 22 atomes de carbone ou des restes alcényles comportant 2 à 22 atomes de carbone, |
| $R^6$ | = -O- ou le reste $-NR^8$, $R^8$ = un reste alcoyle ou hydroxyalcoyle comportant 1 à 4 atomes de carbone, ou un reste d'hydrogène, |
| x | = 2 à 4, |
| M | = un reste hydrocarbure bivalent comportant au moins 4 atomes de carbone, qui possède un groupe hydroxyle et qui peut être interrompu par un atome d'oxygène, |
| n | = un nombre valant de 0 à 200, |
| $X^{\ominus}$ | = un anion inorganique ou organique, |

caractérisé en ce qu'on fait réagir des composés répondant à la formule générale :

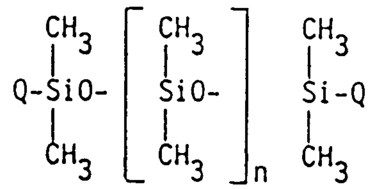

où

Q est un reste hydrocarbure comportant au moins 4 atomes de carbone, qui peut comporter un groupe époxyde et être interrompu par un atome d'oxygène,

n est défini comme ci-dessus,

avec des amines tertiaires répondant à la formule générale:

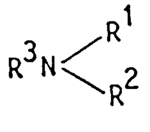 ou 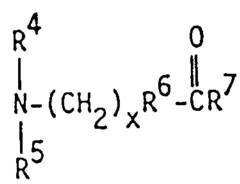 ,

où $R^1$, $R^2$, $R^3$, $R^4$, $R^5$, $R^6$, $R^7$ et x sont définis comme ci-dessus, d'une manière connue en soi, en utilisant des rapports quantitatifs tels qu'à chaque groupe époxyde correspond au moins un groupe amino tertiaire, et on effectue la réaction en présence d'un équivalent d'acide HX, par rapport à l'atome d'azote à quaterniser, et à des températures comprises entre 40 et 120 ° C.

**2.** Utilisation des composés selon la revendication 1 dans des préparations cosmétiques, notamment dans des préparations pour le soin des cheveux.